(19) Europäisches Patentamt
European Patent Office
Office européen des brevets

(11) Veröffentlichungsnummer: **0 255 464**
**B1**

# (12) EUROPÄISCHE PATENTSCHRIFT

(45) Veröffentlichungstag der Patentschrift: **16.08.90**

(51) Int. Cl.$^5$: **C 07 J 53/00,** A 61 K 31/585

(21) Anmeldenummer: **87730047.5**

(22) Anmeldetag: **05.05.87**

(54) **2,2;6,6-Diethylen-3-oxo-17alpha-pregn-4-en-21, 17-carbolactone, Verfahren zu deren Herstellung und diese enthaltende pharm. Präparate.**

(30) Priorität: **07.05.86 DE 3615376**

(43) Veröffentlichungstag der Anmeldung:
**03.02.88 Patentblatt 88/05**

(45) Bekanntmachung des Hinweises auf die Patenterteilung:
**16.08.90 Patentblatt 90/33**

(84) Benannte Vertragsstaaten:
**AT BE CH DE ES FR GB GR IT LI LU NL SE**

(56) Entgegenhaltungen:
**EP-A-0 150 157**
**US-A-3 422 097**

**CHEMICAL ABSTRACTS, Band 103, Nr. 25, 23. Dezember 1985, Seite 930, Zusammenfassung Nr. 215646w, Columbus, Ohio, US; & HU-A-34 994 (GYOGYSZERKUTATO INTEZET) 28-05-1985**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Bittler, Dieter**
**Bölkauer Pfad 11**
**D-1000 Berlin 27 (DE)**
Erfinder: **Nickisch, Klaus, Dr.**
**Alt-Lichtenrade 11**
**D-1000 Berlin 49 (DE)**
Erfinder: **Laurent, Henry, Dr.**
**Glambecker Weg 21**
**D-1000 Berlin 28 (DE)**
Erfinder: **Wiechert, Rudolf, Prof.**
**Petzower Strasse 8a**
**D-1000 Berlin 39 (DE)**
Erfinder: **Haberey, Martin, Dr.**
**Neckarsulmer Strasse 15**
**D-1000 Berlin 46 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 255 464 B1

**Beschreibung**

Die Erfindung betrifft 2,2;6,6-Diethylen-3-oxo-17α-pregn-4-en-21,17α-carbolactone der allgemeinen Formel I

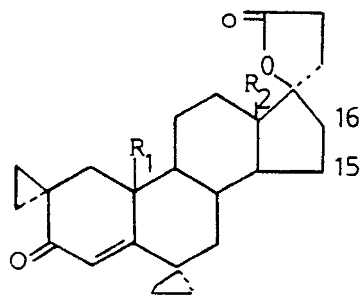

(I),

worin

$R_1$ ein Wasserstoffatom oder eine Methylgruppe,

$R_2$ eine Methyl- oder Ethylgruppe und

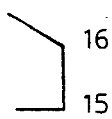 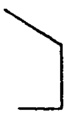 , 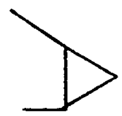 oder 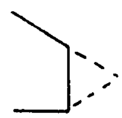

bedeuten,

Verfahren zu deren Herstellung und diese enthaltende pharmazeutische Präparate.

Zur Behandlung von bestimmten Formen der Hypertonie, von Ödemen, des primären Aldosteronismus und anderen durch Aldosteron bedingten endokrinologischen Störungen und als Diurektika werden Substanzen eingesetzt, die die Wirkung von Aldosteron oder Desoxycorticosteron auf die Natrium- und Kaliumsalzausscheidung umkehren und dessen bekanntester Vertreter das schon lange als Handelsprodukt angebotene Spironolacton ist. Bei der Behandlung mit Spironolacton treten aber häufig unerwünschte endokrine Nebenwirkungen auf, die durch die antiandrogene und gestagne Aktivät von Spironolacton hervorgerufen werden. So beobachtet man bei länger andauernder Behandlung von männlichen Patienten mit Spironolacton das Auftreter, von Gynäkomastie (Smith, W. G., The Lancet 1962, S. 886; Mann, N. M., JAMA 1963, S. 778; Clark, E., JAMA 1965, S. 157; Greenblatt, D. J., JAMA 1973, S. 82), und Impotenz (Greenblatt, D. J., JAMA 1973, S. 82) was auf die antiandrogene Nebenwirkung dieses Wirkstoffes zurückgeführt wird (Steelman, S. L. et al., Steroids 1963, S. 449; Schane, H. P., J. of Clinical Endocrinology and Metabolism 1978, S. 691).

Die bei Frauen unter der Behandlung mit Spironolacton auftretenden Nebenerscheinungen wie Amenorrhoe und Cyclusunregelmäßigkeiten werden dagegen der gestagenen Nebenwirkung von Spironolacton angelastet. Beide Nebenwirkungen lassen sich sowohl in Tierversuchen als auch in vitro durch den Rezeptorbindungstest mit dem Androgen- bzw. Gestagen-Rezeptor nachweisen. Das dem Spironolacton in seiner Antialdosterawirkung überlegenes Spirorenon bindet auch noch relativ stark an den Gestagen-Rezeptor.

Es war Aufgabe der vorliegenden Erfindung, Verbindungen zur Verfügung zu stellen, die dem Spirorenon in der Antialdosteronwirkung überlegen sind, aber eine starke vermindet gestagene Nebenwirkung haben.

Im US-Patent 3,422,097 werden 6,6-Ethylen-3-oxo-17α-pregn-4-en-21,17α-carbolactone mit einer Einfach- oder Doppelbindung in 1,2-Stellung beschrieben, wahren in CA: 103,25,1985, 215646w 2,2-Ethylen-6,7-methylen-spirolactone genannt werden.

Aus der deutschen Offenlegungsschrift 34 02 329 sind 6,6-Ethylen-15,16-methylen-3-oxo-17α-pregn-4-21,17-carbolactone bekannt, die sowohl eine Aldosteron-antagonistische als auch eine gestagene Wirkung besitzen.

Die neuen Verbindungen der allgemeinen Formel I weisen eine deutliche Steigerung der antimineralcorticoiden Wirkung auf. Die Affinität der neuen Verbindungen zum Gestagen-Rezeptor wird durch den zusätzlichen Cyclopropyl-Ring in 2-Stellung erheblich gesenkt (der Kompetitionsfaktor ist größer als 20). Die neuen Verbindungen erweisen sich gegenüber Spironenon bei einer bis zu 50% besseren Aldosteron-antagonistischen Wirkung und bei einer bis zu 10 mal geringeren Affinität zum Gestagenrezeptor (gegenüber Dihydrospirorenon, dem aktiven Metaboliten des Spiroenons) als geeignete Antimineralcorticoide.

In der nachfolgenden Tabelle sind die relativen Werte der Antialdosteron-Wirkungsstärke und die Kompetitionsfaktoren in Gestagen-Rezeptor-Test (KG) vom Spirorenon (A) und den erfindungsgemäßen Verbindungen am Beispiel von 2,2;6,6-Diethylen-18-methyl-15β,16β-methylen-3-oxo-19-nor-17α-pregn-4-en-21,17-carbolacton (B) zusammengestellt.

Die Formulierung der pharmazeutischen Präparate auf Basis der neuen Verbindung erfolgt in an sich bekannter Weise, indem man den Wirkstoff mit den in der Galenik gebräuchlichen Trägersubstanzen, Verdünnungsmitteln, gegebenenfalls Geschmackskorrigentien usw. verarbeitet und in die gewünschte Applikationsform überführt.

Für die bevorzugte orale Applikation kommen insbesondere Tabletten, Dragées, Kapseln, Pillen, Suspensionen oder Lösungen infrage.

Fur die parenterale Applikation sind insbesondere ölige Lösungen, wie zum Beispiel Lösungen in Sesamöl, Rizinusöl und Baumwollsamenöl geeignet. Zur Erhöhung der Lösunglichkeit können Lösungsvermittler, wie zum Beispiel Benzylbenzoat oder Benzylalkohol, zugesetzt werden.

Die Herstellung der erfindungsgemäßen Verbindungen der allgemeinen Formel I

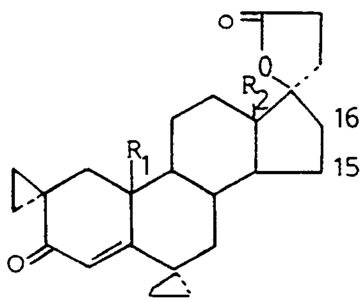

(I),

worin

$R_1$ ein Wasserstoffatom oder eine Methylgruppe,

$R_2$ eine Methyl- oder Ethylgruppe und

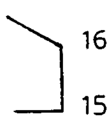 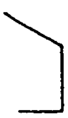 , 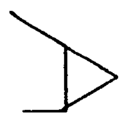 oder 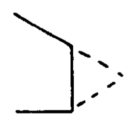

bedeuten, ist dadurch gekennzeichnet, daß man in 3-Oxo-17α-pregn-4-en-21,17-carbolactone der allgemeinen Formel II,

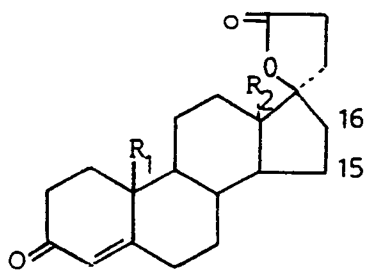

(II),

worin

$R_1$, $R_2$ und

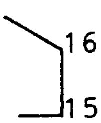

die in Formel I angebenen Bedeutungen haben, in an sich bekannter Weise über die 3,5-Dien-3-amine mit Formalin unter Rückbildung des 4-En-3-oxo-Systems in 2- und 6-Stellung eine Hydroxymethylengruppe einführt, aus den Hydroxymethylgruppen Alkyl- oder Arylsufonyloxymethyl-Gruppen bildet und diese zu Ethylengruppen methyleniert.

Zunachst wird das $\Delta^4$-3-Keton mit einer sekundären Base in das entsprechende $\Delta$-$^{3,5}$-3-Amin überführt.

Als sekundäre Basen sind zum Beispiel Diethylamin, Anilin, Pyrrolidin und Morpholin geeignet.

Zur Einführung der Hydroxymethylgruppen in 2,6-Stellung wird das $\Delta$-$^{3,5}$-3-Amin in alkolischer Lösung mit Formalin behandelt.

Aus der 2,6-Dihydroxymethylverbindung wird mit einem Alkan- oder Arensulfonylchlorid in. Gegenwart einer tert. Base wie Triethylamin in $CH_2Cl_2$ die entsprechende 2,6-Disulfonyloxyverbindung hergestellt.

Die Methylenierung dieser Verbindung zur 2,2;6,6-Diethylenverbindung erfolgt mit Dimethylsulfoxoniummethylid. Hierzu wird das 6-Methylen-Steroid zu einer Suspension von Trimethylsulfoxoniumjodid mit Natriumhydrid in Mineralöl und Dimethylsulfoxid oder zu einer Lösung von

Trimethylsulfoxoniumjodid and Natriumhydroxid in Dimethylsulfoxid gegeben. Die Reaktion ist nach 15 bis 60 Minuten bei 20—40°C beendet.

Beispiel 1

a) Eine Lösung von 3.85 g 18-Methyl-15β,16β-methylen-3-oxo-19-nor-17α-pregn-4-en-21,17-carbolacton in 77 ml Methanol wird mit 1.93 ml Pyrrolidon 15 min unter Rückfluß erhitzt. Nach dem Abkühlen im Eisbad wird der ausgefallene Niederschlag abgesaugt, mit wenig Methanol gewaschen und getrocknet. Es werden 3.75 g 18-Methyl-15β,16β-methylen-3-pyrrolidino-19-nor-17α-pregna-3,5-dien-21,17-carbolacton erhalten.

UV: $\varepsilon_{276}$ = 23 000.

b) Zu einer Suspension von 3.75 g 18-Methyl-15β,16β-methylen-3-pyrrolidino-19-nor-17α-pregna-3,5-dien-21,17-carbolacton in 60 ml Toluol wird innerhalb 30 min bei Raumtemperatur eine Lösung von 3.75 ml 37% iger Formalinlösung in 60 ml Ethanol zugetropft. Nach einer Reaktionszeit von 30 min wird die Reaktionslösung im Vakuum zur Trockne eingedampft. Der Rückstand wird an Kieselgel chromatographiert und es werden 800 mg 2β,6β-Dihydroxymethyl-18-methyl-15β,16β-methylen-3-oxo-19-nor-17α-pregn-4-en-21,17-carbolacton als Öl erhalten.

UV: $\varepsilon_{243}$ = 13 400.

c) 800 mg 2β,6β-Dihydroxymethyl-18-methyl-15β,16β-methylen-3-oxo-19-nor-17α-pregn-4-en-21,17-carbolacton werden in 8 ml Dichlormethan gelöst und im Eisbad abgekühlt. Dann gibt man nacheinander 1.25 ml Triethylamin und 0.475 ml Methansulfonsäurechlorid hinzu, rührt 45 min unter Kühlung, versetzt dann mit 0.225 ml Wasser und rührt weitere 30 min. Die Reaktionslösung wird mit Ether verdünnt, mit Wasser gewaschen, über Natriumsulfat getrocknet und eingedampft. Es werden 1.1 g 2β,6β-Dimesyloxymethyl-18-methyl-15β,16β-methylen-3-oxo-19-nor-17α-pregn-4-en-21,17-carbolacton als Öl erhalten.

d) 3.46 g Trimethylsulfoxoniumiodid werden mit 573 mg Natriumhydrid (55%iger Ölsuspension) in 66 ml Dimethylsulfoxid 1.5 h bei Raumtemperatur gerührt. Zu dieser Lösung werden unter Argon 1.1 g 2β,6β-Dimesyloxy-18-methyl-15β,16β-methylen-3-oxo-19-nor-17α-pregn-4-en-21,17-carbolacton gegeben, anschließend wird 1 h bei Raumtemperatur gerührt. Die Reaktionslösung wird mit Ether verdünnt, mit Wasser gewaschen, getrocknet und eingedampft. Der Rückstand wird an Kieselgel chromatographiert und es werden nach dem Verreiben mit Diisopropylether 200 mg 2,2;6,6-Diethylen-18-methyl-15β,16β-methylen-3-oxo-19-nor-17α-pregn-4-en-21,17-carbolacton vom Schmelzpunkt 213.7°C erhalten.

UV: $\varepsilon_{251}$ = 14 200.

Beispiel 2

a) 5.0 g 15β,16β-Methylen-3-oxo-17α-pregn-4-en-21,17-carbolacton werden in 37.5 ml Methanol mit 2.5 ml Pyrrolidon, wie im Beispiel 1a) beschrieben, umgesetzt und aufgearbeitet. Es werden 5.1 g 15β,16β-Methylen-3-pyrrolidino-17α-pregna-3,5-dien-21,17-carbolacton vom Schmelzpunkt 236—238°C (unter Zersetzung) erhalten.

b) 5.1 g 15β,16β-Methylen-3-pyrrolidino-17α-pregna-3,5-dien-21,17-carbolacton werden in 51 ml Benzol und 102 ml Ethanol mit 5.1 ml 37%iger Formalinlösung, wie im Beispiel 1b) beschrieben, umgesetzt und aufgearbeitet. Es werden nach Chromatographie an Kieselgel 1.02 g 2β,6β-Dihydroxymethyl-15β,16β-methylen-3-oxo-17α-pregn-4-en-21,17-carbolacton als Öl erhalten.

UV: $\varepsilon_{242}$ = 12 600.

c) 1.02 g 2β,6β-Dihydroxymethyl-15β,16β-methylen-3-oxo-17α-pregn-4-en-21,17-carbolacton werden in 10 ml Pyridin mit 1.4 g p-Toluolsulfonsäurechlorid 16 h bei Raumtemperatur stehengelassen. Es werden dann 0.132 ml Wasser zugesetzt, 1 h bei Raumtemperatur gerührt und die Reaktionslösung in Eiswasser eingerührt. Der ausgefallene Niederschlag wird abfiltriert, mit Wasser gewaschen und in Methylenchlorid aufgelöst. Die organisch Phase wird mit Wasser gewaschen, getrocknet und eingedampft. Als Rückstand werden 1.06 g öliges 15β,16β-Methylen-3-oxo-2β,6β-ditosyloxy-17α-pregn-4-en-21,17-carbolacton erhalten.

d) 1.04 g 15β,16β-Methylen-3-oxo-2β,6β-ditosyloxy-17α-pregn-4-en-21,17-carbolacton werden in 50 ml Dimethylsulfoxid mit 2.61 g Trimethylsulfoxoniumiodid und 432 mg Natriumhydrid (55%ige Ölsuspension), wie im Beispiel 1d) beschrieben, umgesetzt und aufgearbeitet. Nach Chromatographie an Kieselgel und Diisopropyletherverreibung werden 245 mg 2,2;6,6-Diethylen-15β,16β-methylen-3-oxo-17α-pregn-4-en-21,17-carbolacton vom Schmelzpunkt 229°C erhalten.

UV: $\varepsilon_{252}$ = 13 100.

Beispiel 3

a) Zu einer Lösung von 12.0 g 15β,16β-Methylen-19-nor-3-oxo-17α-pregn-4-en-21,17-carbolacton in 100 ml Methanol gibt man 6 ml Pyrrolidin und erhitzt 20 min unter Rückfluß. Nach dem Abkühlen wird der ausgefallene Niederschlag abgesaugt, mit wenig kaltem Methanol gewaschen und im Vakuum getrocknet.

Man erhalt 13.2 g 15β,16β-Methylen-19-nor-3-pyrrolidino-17α-pregna-3,5-dien-21,17-carbolacton.

b) Zu einer Lösung von 13.0 g 15β,16β-Methylen-19-nor-3-pyrrolidino-17α-pregna-3,5-dien-21,17-carbolacton in 200 ml Toluol wird innerhalb 1 h bei Raumtemperatur 20 ml 37%ige Formalinlösung in 200 ml Ethanol zugetropft. Nach einer Reaktionszeit von 30 min wird die Reaktionslösung im Vakuum zur Trockne eingedampft. Der erhaltene Rückstand wird an Kieselgel chromatographiert. Man erhält 2.6 g 2β,6β-Dihydroxymethyl-15β,16β-methylen-19-nor-3-oxo-19-nor-17α-pregn-4-en-21,17-carbolacton als Öl.

UV: $\varepsilon_{242}$ = 12 950.

c) Eine Lösung von 2.5 g 2β,6β-Dihydroxymethyl-15β,16β-methylen-3-oxo-19-nor-17α-pregn-4-en-21,17-carbolacton in 25 ml abs. Dichlormethan werden unter Eiskühlung mit 4 ml Triethylamin und 1.5 ml Methansulfonsäurechlorid versetzt und 30 min nachgerührt. Anschließend versetzt man mit 1 ml Wasser und rührt weitere 30 min. Die Reaktionslösung wird dann mit Ether verdünnt, mit Wasser gewaschen, über Natriumsulfat getrocknet und im Vakuum eingedampft. Man erhält 3.05 g 2β,6β-Dimesyloxymethyl-15β,16β-methylen-3-oxo-19-nor-17α-pregn-4-en-21,17-carbolacton als Öl.

d) Eine Lösung von 10.5 g Trimethylsulfoxoniumiodid in 100 ml DMSO wird mit 1.7 g Natriumhydrid (55%ige Ölsuspension) versetzt und 1.5 h bei Raumtemperatur gerührt. Zu dieser Lösung gibt man 3.0 g 2β,6β-Dimesyloxymethyl-15β,16β-methylen-3-oxo-19-nor-17α-pregn-4-en-21,17-carbolacton in 25 ml DMSO und rührt 1 h nach. Die Reaktionslösung wird mit Ether verdünnt, mit Wasser gewaschen, getrocknet und im Vakuum eingedampft. Der Rückstand wird an Kieselgel chromatographiert. Man erhält 720 mg 2,2;6,6-Diethylen-15β,16β-methylen-3-oxo-19-nor-17α-pregn-4-en-21,17-carbolacton.

UV: $\varepsilon_{252}$ = 14 750.

TABELLE

| Verbindung | relative Antialdo-steronwirkung | Kompetitions-faktor $K_G$ |
|---|---|---|
| A | 1 | 2,7 |
| B | 1,5 | 20 |

Die Testmodelle werden ausführlich in den DE—OS'en 3227598 und 3402329 beschrieben.

**Patentansprüche**

1. 2,2;6,6-Diethylen-3-oxo-17α-pregn-4-en-21,17-carbolactone der allgemeinen Formel I,

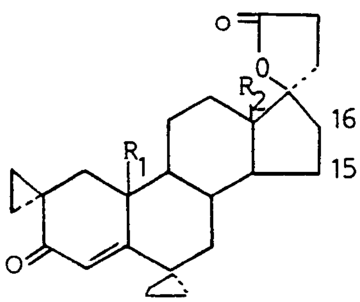

(I),

worin

$R_1$ ein Wasserstoffatom oder eine Methylgruppe,

$R_2$ eine Methyl- oder Ethylgruppe und

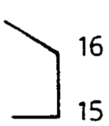 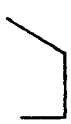, 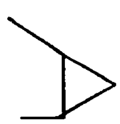 oder 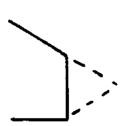

2. 2,2;6,6-Diethylen-18-methyl-15β,16β-methylen-3-oxo-19-nor-17α-pregn-4-en-21,17-carbolacton.

3. 2,2;6,6-Diethylen-15β,16β-methylen-3-oxo-17α-pregn-4-en-21,17-carbolacton.

4. 2,2;6,6-Diethylen-15β,16β-methylen-3-oxo-19-nor-17α-pregn-4-en-21,17-carbolacton.

5. Pharmazeutische Präparate, gekennzeichnet durch den Gehalt an Verbindungen gemäß Anspruch 1 bis 4.

6. Verfahren zur Herstellung von 2,2;6,6-Diethylen-3-oxo-17α-pregn-4-en-21,17-carbolactone der allgemeinen Formel I

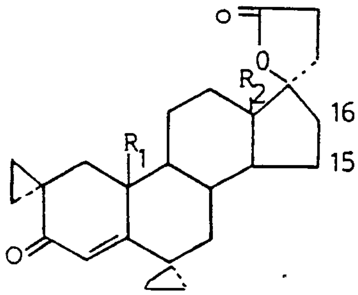

(I),

worin
$R_1$ ein Wasserstoffatom oder eine Methylgruppe,
$R_2$ eine Methyl- oder Ethylgruppe und

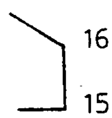 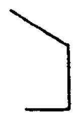 , 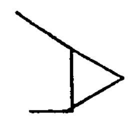 oder 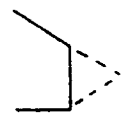

bedeuten, dadurch gekennzeichnet, daß man in 3-Oxo-17α-pregn-4-en-21,17-carbolactone der allgemeinen Formel II,

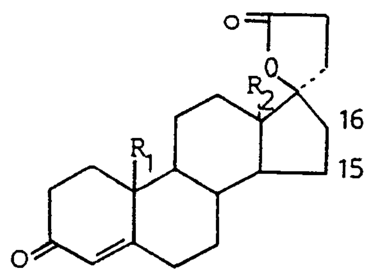

(II),

worin
$R_1$, $R_2$ und

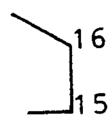

die in Formel I angebenen Bedeutungen haben, in an sich bekannter Weise über die 3,5-Dien-3-amine mit Formalin unter Rückbildung des 4-En-3-oxo-Systems in 2- und 6-Stellung eine Hydroxymethylengruppe einführt, aus den Hydroxymethylgruppen Alkyl- oder Arylsulfonyloxymethyl-Gruppen bildet und diese zu Ethylengruppen methyleniert.

**Revendications**

1. Diéthylène-2,2;6,6-oxo-3-17α-prégnène-4 carbolactones-21,17 répondant à la formule générale I

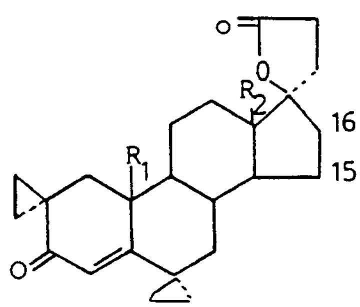

(I),

dans laquelle

$R_1$ représente un atome d'dydrogène ou un radical méthyle,

$R_2$ représente un radical méthyle ou éthyle et

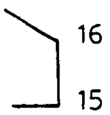 représente 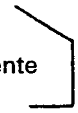 , 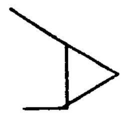 ou 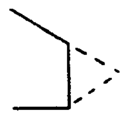

2. Diéthylène-2,2;6,6 méthyl-18 méthylène-15β,16β oxo-3 nor-19 17α-prégnène-4 carbolactones-21,17.

3. Diéthylène-2,2;6,6 méthylène-15β,16β oxo-3 17α-prégnène-4 carbolactones-21,17.

4. Diéthylène-2,2;6,6 méthylène-15β,16β oxo-3 nor-19 17α-prégnène-4 carbolactones-21,17.

5. Compositions pharmaceutiques caractérisées en ce qu'elles contiennent das composés selon l'une quelconque des revendications 1 à 4.

6. Procédé pour préparer des diéthylène-2,2;6,6 oxo-3 17α-prégnène-4 carbolactones-21,17 répondant à la formule générale I

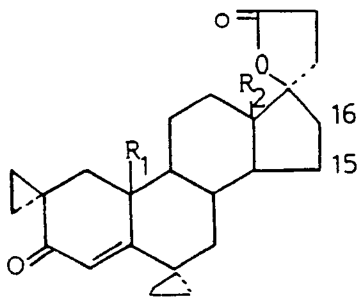

(I),

dans laquelle

$R_1$ représente un atome d'hydrogène ou un radical méthyle,

$R_2$ représente un radical méthyle ou éthyle et

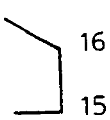 représente 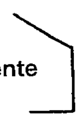 , 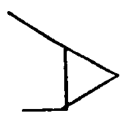 ou 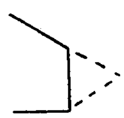

procédé caractérisé en ce que, dans des oxo-3 17α-prégnène-4 carbolactones-21,17 répondant à la formule générale II

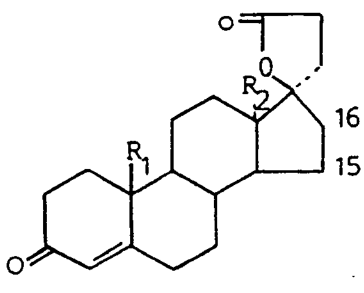

(II),

dans laquelle $R_1$, $R_2$ et

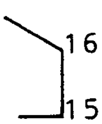

ont les significations qui leur one été donnés à propos de la formule I, on introduit de manière connue, en passant par les diène-3,5 amines-3, par réaction avec le formaldéhyde accompagnée de la régénération du système ène-4 oxo-3, un radical hydroxyméthyle dans chacune des positions 2 et 6, on transforme les radicaux hydroxyméthyles en radicaux alcane-sulfonyl-oxyméthylesouarène-sulfonyloxyméthyles, et on méthylène ces derniers pour les convertir en radicaux éthylènes.

**Claims**

1. 2,2;6,6-diethylene-3-oxo-17α-pregn-4-ene-21,17-carbolactones of general formula I,

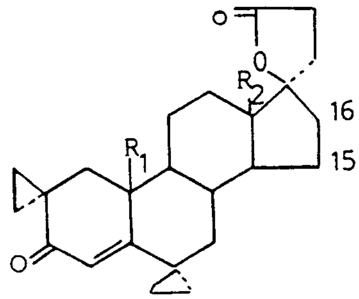

(I),

wherein
$R_1$ represents a hydrogen atom or a methyl group,
$R_2$ represents a methyl or ethyl group and

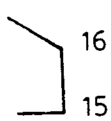 represents 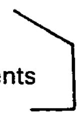 , 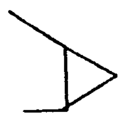 or 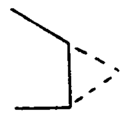

2. 2,2;6,6-diethylene-18-methyl-15β,16β-methylene-3-oxo-19-nor-17α-pregn-4-ene-21,17-carbolactone.

3. 2,2;6,6-diethylene-15β,16β-methylene-3-oxo-17α-pregn-4-ene-21,17-carbolactone.

4. 2,2;6,6-diethylene-15β,16β-methylene-3-oxo-19-nor-17α-pregn-4-ene-21,17-carbolactone.

5. Pharmaceutical preparations, characterised in that they contain compounds according to claims 1 to 4.

6. Process for the preparation of 2,2;6,6-diethylene-3-oxo-17α-pregn-4-ene-21,17-carbolactones of general formula I,

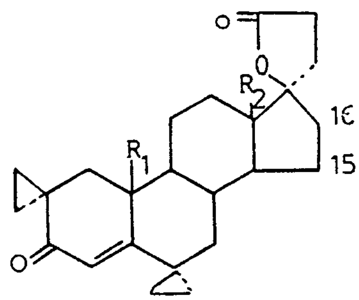

(I),

wherein
$R_1$ represents a hydrogen atom or a methyl group,
$R_2$ represents a methyl or ethyl group and

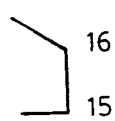 represents 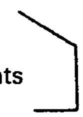 , 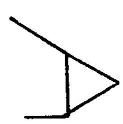 or 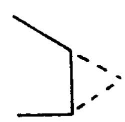

characterised in that in a manner known *per se* a hydroxymethylene group is introduced, in the 2- and 6-positions, into 3-oxo-17α-pregn-4-ene-21,17-carbolactones of general formula II

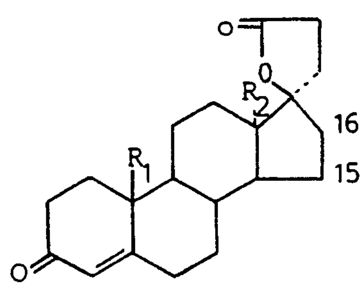

(II),

wherein $R_1$, $R_2$ and

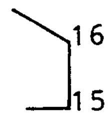

have the meanings indicated for formula I, via the 3,5-diene-3-amines with reformation of the 4-ene-3-oxo system with formalin, alkyl- or aryl-sulphonyloxymethyl groups are formed from the hydroxymethyl groups, and those groups are methylenated to form ethylene groups.